# EUROPEAN PATENT SPECIFICATION

(11) **EP 0 394 319 B1**
(45) Date of publication and mention of the grant of the patent: **15.12.1993**
(21) Application number: 89900662.1
(22) Date of filing: 16.12.1988
(51) Int. Cl.: A23L 3/3463, A23L 3/3499, A23L 3/3526, B65B 55/12, A01N 33/24

(54) **CONTROL OF BIOLOGICAL CHARACTERISTICS OF MATERIAL**
STEUERUNG DER BIOLOGISCHEN KENNZEICHEN EINES MATERIALS
CONTROLE DES CARACTERISTIQUES BIOLOGIQUES D'UN MATERIAU

(30) Priority: 18.12.1987 US 135004
(43) Date of publication of application: 31.10.1990
(73) Proprietor: TIEDEMANNS-JOH. H. ANDRESEN ANS, N-0655 Oslo 6 (NO); REPLIGEN CORPORATION, Cambridge, MA 02139 (US)
(72) Inventor: WITT, Daniel, P., South Hamilton, MA 01982 (US); DOTEN, Reed, Charlestown, MA 02129 (US); LAI, Chee, Kong, Littleton, MA 01460 (US); VATNE, Kaare, N-1396 Bjorkas (NO); ULRICHSEN, Borre, B., N-0376 Oslo 3 (NO); CASTBERG, Helge, B., N-1410 Kolbotn (NO)
(74) Representative: Burrows, Anthony Gregory
(86) International application number: NO8800097
(87) International publication number: WO8905762

(56) References cited:
- EP-A- 40 740
- APPLIED AND ENVIRONMENTAL MICROBIOLOGY, vol. 53, no.9, June 1987, Y. ENDO et al., "Antimicrobial activity of tertiary amine covalently bonded to a polystyreme fiber", pages 2050-2054
- DIALOG INFORMATIONAL SERVICES, File 51 ; Food Science and Technology, Abstracts 69-89, accession no.229963, Y. NAKAGAWA et al."Antimicrobial characteristic of insoluble alkylpyridinium iodide" & APPLIED AND ENVIRONMENTAL MICROBIOLOGY, 1982, 43(5), 1041-50

## Description

### TECHNICAL FIELD

This invention relates to a method for controlling at least one biological characteristic of a material. The invention also relates to packaging material which can perform the control and to a controlling substance.

### BACKGROUND ART

Examples of the detrimental effect of micro-organisms in industry, health and personal care as well as in agriculture are numerous.

Spoilage of foods normally arises as a result of the action of micro-organisms, either bacteria, fungi, or both, on food components. Process developments have included ultrahigh temperature (UHT) treatment of milk which can produce a product with a shelf life of several months without refrigeration. This process, however, relies on treatment with higher temperatures (above 130°C) than pasteurization and results in significant alterations in the organoleptic properties of treated milk. Many consumers complain of a "cooked" flavour of milk when UHT-treated. They also complain of less fresh taste in fruit juices heat-treated at higher temperatures.

Most spoilage of packaged products arises from micro-organisms introduced after the heat treatment.

Today food products are handled in a series of steps from the harvest of raw materials until they are being packaged or processed to products. In many countries the hygienic standard within the food industry is low. It may therefore be advantageous to insert a system for inhibiting microbial growth at a number of points in the processing chain in order to ensure a high quality of the finished products.

It would be desirable to develop a general method for the control of microbial growth and/or metabolism in raw material, during processing, and in packaged foods. Desirable features of such a method would be to avoid additive steps so as not to materially alter the nature of the food and to make the control method an active property of the material packaging the food.

It is known that certain proteins can bind micro-organisms such as bacteria.

For example, International Patent Application Publication Number W082/04264 discloses a method for rapid determination of biochemical data for micro-organisms,in which they are bound to an adsorbent, usually a carrier in the form of gel, provided with antibodies corresponding to the micro-organisms and a nutritient medium is added in order to start the metabolism of the cell. The method can also be used for the determination of the concentration of biochemical substances, which influence the metabolism of the cell, for instance vitamins and antibiotica.

As another example, the binding properties of lectins are detailed in the article "Lectins as Molecules and Tools" by HALINA LIS and NATHAN SHARON in Ann. Rev. Biochem. 1986, No. 55, pages 35-67.

As a further example, DE-A-3523615 discloses medical equipment, in particular cannula, catheters or implants of plastics or plastic-coated metal, in which the plastics surface is covered with a layer of gammaglobulins. The disclosure states that, by means of appropriate covering of such medical equipment with layers of gammaglobulins, it is possible to achieve antigen-antibody complex formation on the surface of the plastics in vivo. This means that germs on contact with the gammaglobulins by virtue of the antigenic properties are bound and their mobilization is inhibited. The antibodies or gammaglobulins are firmly bound to the plastics surface and not given off into the medium.

However, no commercially practical method applicable to the removal or inhibition of micro-organisms in food is disclosed in any of those three documents.

It is also known that certain proteins can destroy micro-organisms, such as spores.

For example, the use of nisin as a microbiocidal additive in processed foods is disclosed in a paper by K. C. EAPEN et al. in the Journal of the Association of Food Scientists & Technologists, India, Volume 20 No. 5, Sept./ Oct. 1983, pages 231-240.

However, the use of such additive in processed food is becoming increasingly controversial, with manufacturers avoiding such use where reasonably possible.

Several immobilized quaternary ammonium salts have also been shown to have antimicrobial activities and in particular, 3-trimethoxysilyl propylocatdecyl dimethyl ammonium chloride has had widespread use in the textile industry. Endo et al, Applied and Environmental Microbiology, Sept. 1987, p2050-2055 have indicated, for the first time, that a tertiary methyl amine covalently bound to polystyrene fibre (TAF) is antimicrobial in a buffer solution. The mode of action of this tertiary amine fibre appears to involve a binding of microbial cells to the fibre surface followed by an interaction between the tertiary amine group and the cells which produces cell membrane damage, by an unknown mechanism, and ultimately cell lysis. Endo et al, are particularly concerned with covalently bonding tertiary amine to artificial organs to avoid bacterial infection upon insertion of the organs.

Although the original observation by Endo et al that TAF is antimicrobial suggests that other immobilized tertiary amine surfaces may be antimicrobial in a simple salt solution, it does not indicate that such an antimicrobial surface will also be active in a complex medium such as milk or fruit juice. For example, the antimicrobial activity of immobilized quaternary ammonium salts, which is quite high in water or simple salt solutions, is blocked or diminished by exposure to a variety of complex media. By the term "complex medium" as used herein is meant any medium able to support the growth of micro-organisms with the medium having the following characteristics: the carbon and nitrogen sources present for growth are not solely simple carbohydrates and amino acids but are a mixture of compounds, which compounds are, for example, carbohydrates, polysaccharides, proteins, peptides, lipids, organic acids, vitamins and cofactors present as, or derived from, nutrient rich, undefined compositions, for example plant and animal produced fluids, plant and animal tissue extracts and tissue hydrolysates.

"Amberlite" IRA-68 and IRA-94 beads referred to hereinafter are known to be used for de-ionising of water.

It is also known that, as an alternative to inactivating micro-organisms by heat or other conventional treatment, their inhibition and/or inactivation may be obtained by restricting the supply of essential growth factors. Essential growth factors may be compositions such as trace minerals, vitamins, organic acids, sugars or gases (e.g. oxygen). Which of these factors are essential will depend on the type of micro-organism.

One factor that most, if not all, micro-organisms require from the environment is the element iron. Iron is required as a cofactor in many enzyme systems which the cells use for metabolic processes as well as being absolutely essential for most microbial ribonucleotide reductases.

U.S. Patents 4,530,963 and 4,585,559 to DeVoe et al disclose a method for inhibiting microbial growth in a liquid medium containing ferric iron by contacting the medium with a siderophore covalently bound to an insoluble carrier. DeVoe et al disclose the use of siderophores including deferrioxamine B and deferriferrichrome which are not practically effective for limiting microbial growth since the siderophores employed have a kinetic exchange rate for iron which results in bound iron being subsequently released from the siderophore into the medium being treated.

In liquids with low pH and reducing environments, such as fruit juices, almost all iron will be in the ferrous form.

DE-A-2922347 discloses a process for the manufacture of materials with anti-microbial properties, in a very wide range of fields. The disclosure states that an antimicrobially effective substance can be grafted onto the surface of a material with formation of a true chemical bond and, in particular, the thus grafted and chemically fixed substance retains its antimicrobial activity. Thereby, such substances, fixed by covalent chemical bonds, are not only secured against detachment, but are also capable of inhibiting the growth of micro- organisms encountered by either killing them or limiting their growth, and in this manner keep their environment free from germs. Moreover, they are capable of protecting the substrate material against microbial corrosion. A number of methods are given for fixing the substances to solids or solid surfaces so as to manufacture the materials with antimicrobial properties. One method is the treatment of the solid so as to create reactive surface groups, insofar as it does not inherently possess them, such as does cellulose in the form of hydroxyl groups, and fixing of a coupling comment with at least two reactive functional groups.

The antimicrobially effective substance must have, besides its antimicrobially effective group(s), at least one reactive group to couple to the unreacted functional group(s) of the coupling component. A suitable coupling reactive group of an anti-microbial compound is, for example, an amino group. In another method, starting out from an antimicrobially effective substance, which, besides its antimicrobially effective group(s), must have a further functional group, a reactive microbicide is produced by reaction with a bi-functional or multi-functional coupling component. This reactive microbicide is then grafted onto the solid surface in such a manner that the free functional group(s) of the coupling component react with surface groups of the solid with formation of a chemical bond. It is also disclosed that, in the presence of suitable reactive groups on the solid as well as in the antimicrobial substance, a chemical bonding of the antimicrobially effective substance may be possible, under certain circumstances, also without a coupling component. Cellulose, as a result of a special oxidative treatment, possesses very reactive aldehyde surface groups and, in this case, the antimicrobial substance with available amino group is sufficient to achieve chemical bonding. In a further embodiment of the process, and by means of special chemical treatment of the solid, antimicrobially effective surface groups can be created directly. The conversion of the vicinal hydroxyl groups of the cellulose into aldehyde groups through periodate oxidation is referred to. Diverse applications of the process and of the objects manufactured by the process are described. Examples of applications are in the field of hygiene, e.g. the treatment of OP garments, covering sheets in the OP area, dressing materials and, not least, the treatment of filter materials in germ-filtering installations. Among antimicrobially effective materials and objects referred to are fibres, tissues, felts, filters, membranes and articles of clothing, and also objects such as vessels, pipes, and packaging materials for the foodstuffs and pharmaceuticals industries. Reference is also made to materials or objects protected against microbial corrosion such as textiles, timber structures, and electronic and fine-mechanical components. The only example given of a possible solid or solid surface is cellulose. However, cellulose is in fact not practical as a packaging for liquid or moist packaged materials, since contact with liquids causes it to swell and thus become permeable to the liquid. It is also a poor barrier to migration of flavours, etc.

DE-A-2817854 discloses a container for the packaging of perishable materials, especially fruit juices, beer, or non-carbonated beverages, in which the inner surface of the container wall is provided with a polymer coating comprising repetitive functional groups selected from carbonyl, carboxyl, anhydride, epoxy and isocyanate groups, the functional groups comprising covalently bonded biologically active enzymes. The biologically active enzymes may be bonded to the functional groups of the coating by non-essential functional groups which are selected from amino, carboxyl, hydroxyl, phenol, imidazone and hydrosulphide groups. However, the activity of the biologically active enzymes is very liable to be substantially inhibited by constituents (particularly other enzymes) of the perishable materials mentioned.

### DISCLOSURE OF INVENTION

According to one aspect of the present invention, there is provided a method for controlling the growth of micro-organisms in a packaged material in the form of a complex medium and comprising liquid, wherein the material is contacted with a substantially insoluble, immobilized, growth-inhibiting substance at a surface of a packaging material, said substance being of a composition to be active upon said packaged material, characterized in that the at least one active substance is not an enzyme and in that said activity is promoted by producing relative motion between the packaged material and the active substance(s).

According to a second aspect of the present invention, there is provided packaging material having a substantially insoluble, immobilized, growth-inhibiting substance at a surface thereof for contacting another material in the form of a complex medium and comprising liquid, said substance being of a composition to be active upon said other material to inhibit the growth of micro-organisms in said other material, characterized in that there is a plurality of active substances which are not enzymes.

According to a third aspect of the present invention, there is provided packaging material having a substantially insoluble, immobilized, growth-inhibiting substance at a surface thereof for contacting another material in the form of a complex medium and comprising liquid, said substance being of a composition to be active upon said other material to inhibit the growth of micro-organisms in said other material, wherein said active substance(s) comprise(s) an amine-containing compound of which the amine is available for and capable of inflicting decomposed damage upon said micro-organisms in said other material to inhibit their growth in said other material.

According to a fourth aspect of the present invention, there is provided packaging material having a substantially insoluble, immobilized, growth-inhibiting substance at a surface thereof for contacting another material in the form of a complex medium and comprising liquid, said substance being of a composition to be active upon said other material to inhibit the growth of micro-organisms in said other material, and wherein said active substance(s) comprise(s) a binding substance in the form of a binding composition having affinity for at least one trace metal constituting a biological growth factor and providing a binding constant and a kinetic stability for binding said trace metal(s) sufficient for the binding composition successfully to compete for said trace metal(s) with said micro-organisms and to retain the bound trace metal(s) thus restricting the availability of said trace metal(s) to said micro-organisms.

The active substance(s) serve(s) to inhibit the growth of micro-organisms in complex media, such as foods.

The or each active substance is advantageously an inorganic or organic composition which upon binding to the surface retains an affinity for micro-organisms, thereby to bind (and destroy) the micro-organisms.

Said active substance(s) preferably comprise(s) a binding substance having a binding affinity to biological cells in said other material, thereby to bond (and destroy) said micro-organisms, or a binding affinity to at least one biological growth factor of said micro-organisms, thereby to restrict the availability of said growth factor(s) to said micro-organisms.

The said binding substance advantageously has a high binding affinity to said micro-organisms or their growth factor(s) as the case may be, but a low binding affinity to other compositions in the material.

In cases when said binding substance has a binding affinity to said micro-organisms, the substance may be a binding polymer, for example a lectin, an antibody, a polysaccharide, or an enzyme (such as a lysozyme). The binding polymer preferably has a specific affinity to a glycoconjugate or polysaccharide, and is immobilized on a wide variety of surfaces including polymer resins or copolymers such as polystyrene, polyethylene, polypropylene, polyamides, polystyrene-acrylonitrile and the like, paperboard which may or may not contain a polymer, or glass. Typical food containers comprise a polymer, or a metal or paperboard coated with a polymer such as polyethylene. Paperboard food containers typically are coated with or impregnated by a polymer such as polyethylene.

Good contact between liquid material and the active substance(s) is provided by the relative motion between the liquid and the surface coated with the active substance(s) in a distribution container resulting from normal handling of the distribution container. Alternatively, the or each active substance is bound to the surface of a device separate from the container which provides intimate contact with liquid product.

According to a fifth aspect of the present invention, there is provided a method comprising providing a material in the form of a complex medium which may contain micro-organisms, and controlling the growth of said micro-organisms in said complex medium by contacting the complex medium with a surface comprised of an active substance in the form of a binding substance having a binding affinity to said micro-organisms in said complex medium, thereby to bind said micro-organisms, said substance having a high binding affinity for said micro-organisms, but a low binding affinity for constituents in said complex medium other than said micro-organisms, said active substance not being an enzyme.

According to a sixth aspect of the present invention, there is provided in combination, a complex medium, a binding substance having a binding affinity to micro-organisms in said medium for thereby binding said micro-organisms, and a biocidal agent for inactivating said micro-organisms, said binding substance being immobilized at a surface contacting said complex medium.

In this manner, micro-organisms, such as bacteria, in the material can be controlled by immobilizing the micro-organisms. It therefore becomes relatively easy to separate the micro-organisms from the material. While immobilized, the micro-organisms can be subjected to microbiocidal compounds.

Advantageously, a method is provided wherein binding organic compounds, for example binding polymers, bound to an insoluble support are utilized as a means of immobilizing micro-organisms and removing the micro-organisms from a liquid, solid or gaseous phase. The binding polymer is preferably a protein (e.g. lectin, antibody or enzyme) or a polysaccharide which has a specific affinity for a micro-organism. The binding of the polymer to the insoluble support can be either covalent using any of a number of appropriate chemical coupling procedures or non-covalent by taking advantage of the hydrophobic nature of the polymer to promote passive adsorption to a material contact surface. When the appropriate polymers have been identified, this technique can be used directly to remove bacteria from liquid, solid or gaseous material such as bulk milk or to restrict or compartmentalize the bacteria to a solid surface by binding thereto.

In a further application, bacteria can be isolated in a column or a filter, and thus effect decontamination of a gas or liquid before filling it into containers. The filter or column will gradually become saturated and must either be continuously regenerated or replaced.

Examples of proteins which destroy or inhibit micro-organisms and which can be used in conjunction with the binding compounds to control microbial growth are lytic enzymes, such as lysozyme or lysostaphin and bactericidal oxidative enzymes such as lactoperoxidase or myeloperoxidase.

The present method of immobilizing bacteria can also have a number of other applications, such as in industrial fermentation to avoid precipitation, in the filtration of water, or the installation of protein-coated surfaces in the water of swimming pools, to avoid or substantially reduce chlorination. It is also contemplated that a protein-coated filter can be used in mouth binds or face masks, to reduce the risk of infection from bacteria or vires in the air. Another application is the removal of sulphate-reducing bacteria from engine oil or in oil-field applications.

According to a seventh aspect of the present invention, there is provided a method for controlling the growth of micro-organisms in a material in the form of a complex medium, wherein the material is contacted with an active substance, characterized in that the active substance is in the form of an immobilized amine-containing compound, the amine being available for and capable of inflicting sufficient damage upon micro-organisms in said material to inhibit their growth in said material.

According to an eighth aspect of the present invention, there is provided use of an active substance for inflicting sufficient damage upon micro-organisms in a complex medium to inhibit their growth in said medium, characterised in that said active substance comprises an amine-containing compound immobilized at a surface contacting said complex medium.

It thereby becomes possible to inhibit growth of micro organisms in complex media, for example liquid foods such as milk or fruit juice, solid foods such as meat or fish, or contaminated blood or serum, while reducing dependence upon heat or other conventional treatment.

This is based upon the discovery that certain amine-containing compounds which exhibit antimicrobial activity in simple media such as buffer solutions also exhibit antimicrobial activity in complex media such as foods. In particular, it has been found that certain tertiary amine compounds exhibit antimicrobial activity in complex media. This finding is surprising since no tertiary amines have previously been identified that exhibit antimicrobial activity both in simple media such as buffer solutions as well as complex media such as foods.

According to a ninth aspect of the present invention, there is provided a method for controlling the growth of micro-organisms in a material in the form of a complex medium by contacting the complex medium with an active substance in the form of a binding substance comprising a binding composition having affinity for at least one trace metal constituting a biological growth factor of micro-organisms in said complex medium, wherein there is used a binding composition providing a binding constant and a kinetic stability for binding said trace metal(s) sufficient for the binding composition successfully to compete with micro-organisms for said trace metal(s) and to retain the bound trace metal(s), thus restricting the availability of said trace metal(s) to said micro-organisms.

It is thereby possible effectively to control micro-organisms such as bacteria in a material by removing at least one micro-organism growth supporting factor from the material.

Thus the binding between the present binding composition and the growth factor(s) gives a stable complex in which neither of the reactants is substantially decomposed as a result of the affinity nor substantially degraded over reasonable time, i.e. the growth factor(s) form(s) a substantially insoluble complex with the binding composition. For example, by using an immobilized synthetic ferrous chelator of sufficient strength to successfully compete with the micro-organisms for ferrous iron, growth of micro-organisms can be effectively controlled.

Iron, such as ferrous iron, contained in the material can be isolated in a column or filter containing the immobilized binding composition, thus to effect the iron depletion of liquid food material before filling the material in containers. The filter or column will gradually become saturated and is either continuously regenerated or replaced.

The present binding composition and method can also have a number of other applications, such as the filtration of water, or the treatment of non-food liquids by the installation of binding surfaces in containers or process equipment.

### DESCRIPTION OF DRAWING

In order that the invention may be clearly understood and readily carried into effect, three embodiments thereof will now be described, all with reference to the accompanying diagrammatic drawing, which shows a vertical section through a plastics-coated paperboard container used for packaging liquid food.

### MODES FOR CARRYING OUT THE INVENTION

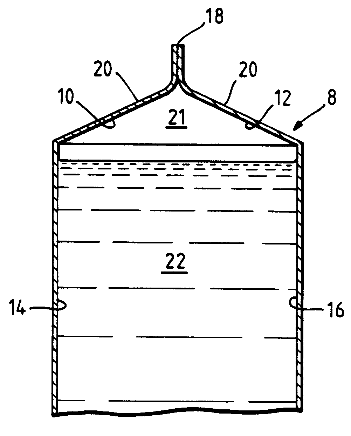

Referring to the drawing, the container 8 is of the character disclosed in United States Patent 4, 211, 357, but has one or more active substance(s) bonded to its inner surfaces 10, 12, 14 and 16. In the case of a plurality of active substances, these can be either bonded to respective zones (which may intermingle) of the surfaces, or mixed together (if compatible) and the mixture bonded to the surfaces. The substance(s) can also be bonded to the inner bottom surface (not shown) of the container 8. The container 8 includes a top seal 18 that closes the roof 20, thereby forming an open space 21 and sealing the liquid food 22 from the outside atmosphere. The substance(s) bound to the inner surfaces 10, 12, 14 and 16 of the container 8 is (are) active upon the liquid food 22. In another example, only the inner surfaces 10 and 12 of the roof 20 are coated with the substance(s), but these inner surfaces 10 and 12 contact the liquid 22 by normal handling of the container 8, or through controlled agitation of the container 8 and liquid 22, or through the container 8 being stored or transported in all inverted condition.

### EMBODIMENT I

This embodiment will be described herein with specific reference to immobilizing bacteria from foods such as milk and fruit-based drinks, including fruit drinks and nectars. However, it is to be understood that the present embodiment is useful for immobilizing bacteria from gaseous, liquid and solid phases in general.

The present embodiment utilizes a matrix to which one or more specific binding polymers have been bound either covalently or passively. Examples of such matrices include polymers, such as polyethylene, or polystyrene; glass and cellulose-based compositions to which the binding polymer has been bound by passive adsorption or by covalent bonding. The binding-polymer-coated surface may be placed in contact with a food product, for example, a liquid product such as milk, either as an in-line cartridge or another contact-enhancing system during a transfer step or as a part of the final packaging material. This serves to remove the contaminating bacteria by adsorption or sequestration to the solid phase with the ultimate result of prolonging the shelf life of the packaged product and/or eliminating undesirable organisms.

If the final packaging material is a distribution container 8, good contact between the liquid product 22 and the binding polymer bound to the matrix is provided by the relative motion between the product 22 and the binding-polymer-covered surface (10, 12, 14, 16) in the container 8 resulting from normal handling of the container. Alternatively, the binding polymer is bound to the surface of a separate device which moves and agitates the liquid product. Separation of the bacteria is obtained when the device is removed or the liquid material 22 is poured out of the container 8, the bacteria remaining bonded to the inner surface (10, 12, 14, 16) of the emptied container.

Binding polymers are polymers such as lectins, polysaccharides, antibodies, or lysozyme. The binding is mediated by means of specific interaction between a molecule exposed on the bacterial cell surface and the binding polymer.

The nature of the immobilization of the binding polymers to a solid support can be either covalent or non-covalent. The important characteristic of this interaction is that the binding polymers remain tightly bound and not be released during the steps of bacterial binding. Covalent binding of the binding polymers can be accomplished by a wide variety of techniques which are well known in the art.

As an alternative to covalent immobilization it may be desirable under some circumstances to bind the binding polymers non-covalently to a material contact surface by hydrophobic interaction. An aqueous solution of a binding polymer is incubated in contact with the material contact surface to which the protein is to be bound. This results in the time, temperature, and concentration dependent formation of a molecular layer of binding polymer on the hydrophobic material contact surface by the process of passive adsorption.

Depending on the micro-organisms present and the parameters of the application, the extraction process could be either selective, to remove a particular unwanted micro-organism species, or non-selective in the sense that most or all contaminating micro-organisms are removed.

The binding polymers that have been tested in the present embodiment have a large capacity for binding to bacteria, either specifically to certain types of bacteria or more non-specifically to several types. In the case of lectins as binding polymers, the binding ceases and is reversed on the addition of competing sugar solutions and therefore binding-polymer-coated surfaces can be "washed" clean in this way.

Certain solid foods such as fish and meat are degraded by bacterial contamination and the spread of contamination from the surface. It is within the scope of this embodiment to apply the same principle for achievement of increased shelf life of such products by surrounding the solid foods with a liquid and a film, or film alone, wherein the binding polymer is bound to the film.

Referring to the container 8 shown in the drawing, binding polymer bonded to inner surfaces of the container 8 functions to bind and immobilize micro-organisms in the liquid 22. If desired an active substance which blocks the metabolism of the micro-organism also can be bound to some or all of the inner surfaces of the container 8.

This embodiment will now be described in greater detail with reference to the following non-limiting examples:

### Example 1

Conditions were evaluated for binding binding polymers to material contact surfaces by passive adsorption. Good results were obtained for several polymers, for example, the lectins from Dolichos biflorus and Vicia villosa. Other lectins including Concanavalin A gave similar results suggesting that this is a common characteristic of lectins. Immunoglobulin molecules possess similar properties as well.

The lectins were radioactively labelled and stock solutions thereof were made in a buffer. These were then serially diluted and aliquots spotted on either a polystyrene or polyethylene surface and incubated for 1 hour at 37°C followed by washing in buffer. The polyethylene surface in these studies was standard milk carton material which consisted of a polyethylene sheet coated over a cardboard surface.

The results demonstrated that the binding approached saturation at 5-10 ng/mm² depending on the lectin tested. We conclude from this that lectins bind to hydrophobic plastics surfaces with the upper limit of binding dictated by the packing density of the hydrated molecules on the surface.

### Example 2

When binding polymers were passively adsorbed to a polymer surface (see Example 1) and then exposed to a suspension of washed human RBCs in buffer, a binding of RBCs to the immobilized protein occurred.

Following a 30 min. incubation of polymer with RBCs the surface of the treated polymer showed by microscopic observation a confluent carpet of cells with most binding polymers tested.

Good results were obtained with a variety of binding polymers including Dolichos biflorus lectin and Vicia villosa lectin.

### Example 3

Three representative milk spoilage bacteria which had been isolated from pasteurized market milk were surveyed for their ability to be bound to a variety of binding polymers passively adsorbed onto a polymer surface. The three organisms were identified as Bacillus brevis, Enterobacter sp. and Pseudomonas sp. by classification methods which are well known in the art. Binding polymers were passively adsorbed to a polymer surface in a fashion similar to that described in the previous examples. the bacteria were suspended in phosphate buffered saline and suspensions of these three organisms were then placed in contact with the treated polymeric surfaces. Binding of the bacteria was evaluated by micro-scopic examination. The results of this survey indicated that several binding polymers such as lectins from Dolichos biflorus, Vicia villosa, Limulus polyphemus and Solanum tuberosum, were effective in binding typical species of these diverse microbial classes.

### Example 4

100 CFU/ml of Bacillus sp. cells were suspended in Tris buffered saline and incubated with a polymer matrix to which the binding polymers had been covalently coupled. The number of free bacteria in suspension was determined by microscopic evaluation of an aliquot of the supernatant fluid (after settling of the matrix). By this method, the ability of the immobilized binding polymers to bind and remove the microbial contamination was demonstrated and the affinity of bacterial cells to binding-polymer-covered matrices was established under the conditions of the experiment. The effectiveness of the bacterial removal by this method using eg. Dolichos biflorus was found to be in some instances almost 100%.

### Example 5

A strain of Saccharomyces was obtained from Pure-Pak, Inc., Walled Lake, MI, United States of America. This strain was originally isolated from cranberry juice and had been found to grow in and spoil a variety of fruit juices.

The cells were grown either in apple juice, which had been sterile filtered, or in synthetic medium provided by Difco.

Similar results were obtained regardless of the growth conditions.

The lectin from Dolichos biflorus was obtained from Sigma Chemical Co. and immobilized either by passive adsorption to a polystyrene surface or by cyanogen bromide coupling to agarose beads.

When Saccharomyces cells were exposed to a surface containing the immobilized lectin, the cells were found to become rapidly and tightly bound to the surface.

For instance at least 90% of the cells in the suspension were found to become bound within 1-2 hours to the insoluble agarose beads carrying the immobilized Dolichos biflorus lectin.

This binding was very stable and was not significantly reversible within 24 hours. Cells which were bound in this fashion, were easily removed from the juice either by allowing the beads to settle and decanting the supernatant or by passage over a macroporous filter such as glass wool.

### Example 6

Solanum tuberosum lectin was bound to a polystyrene Petri plate by passive adsorption and the plate was exposed to milk to which had been added 10⁶ cells/ml of either Bacillus brevus or Pseudomonas fluorescens. After incubation for 30 minutes the plates were rinsed and examined by light microscopy. In both cases, binding of bacteria to the lectin coated surface was noted but the binding area was seen to be substantially less than observed to be covered with vesicles which were identified as milk fat globules. This experiment demonstrates that this lectin, which is capable of binding milk spoilage bacteria, can also bind surface components of the membranes of the milk fat globules and that this latter activity leads to a decrease in the effectiveness of the technique. This decrease in effectiveness rules out the use of this lectin in milk.

### Example 7

A study was conducted to determine the optimal density of binding polymer on the insoluble support matrix. CNBr activated agarose beads were conjugated with varying levels of lectin, from 1 mg/ml up to 50 mg/ml, and exposed to cells for one hour. Efficiency of binding was determined by assessing the number of free cells left in the solution phase. It was found that maximal binding of cells occurred when lectin loads on the beads were between 15 and 30 mg/ml. The binding under these circumstances was in excess of 95%. No additional binding was found at loads of up to 50 mg/ml. A significant decrease in binding was observed with lectin loads of less than 10 mg/ml.

### Example 8

(This Example can also be considered as inserted in Embodiment II).

Binding of Saccharomyces cerevisiae to tertiary amines was shown by two methods. In the first, columns (90 x 6 mm) were packed with IRA-68 and IRA-94 beads. The columns were equilibrated with 1/4 strength Ringers solution. One ml of S. cerevisiae cells (10⁶ cells/ml) suspended in Ringers solution was applied on the column and eluted with Ringers solution. One ml fractions of eluate were counted in a bacterial counting chamber, and the number of cells eluted was calculated. 45% of the cells were retarded in both IRA-68 and IRA-94. In the second method, designed to directly demonstrate binding in solution, IRA-68 beads were equilibrated in Ringers solution and 250 mg beads put in a 10 ml test tube. 2 ml of S. cerevisiae suspended in Ringers solution was transferred to the tube. The test tube was incubated vertically at room temperature for 2 hours, the pH measured (5.4) and the supernatant decanted off. Sterile Ringers solution was added twice to wash away unbound cells. Binding of S. cerevisiae cells to IRA-68 beads was shown by microscopic examination.

### Example 9

The binding ability of various micro-organisms to other selected proteins was investigated. Lysozyme, fibronectin and bovine serum albumine (BSA) were covalently coupled to CNBr-activated agarose beads.

Strong binding (greater than 80% coverage) to lysozyme was demonstrated for Pseudomonas fluorescens TP2, Streptococcus lactis, Streptococcus thermophilus, Staphylococcus xylosus, while Bacillus subtilis vegetative cells bound to a lesser degree. No binding of Saccharomyces cerevisiae, Escherichia coli, Serratia marcescens or B. subtilis spores was detected.

### EMBODIMENT II

This embodiment will be described herein with specific reference to inhibiting microbial growth in foods as a complex medium, such as milk and fruit-based drinks, including fruit drinks and nectars. However, it is to be understood that the present embodiment is useful for inhibiting microbial growth in gaseous, liquid and solid phases in general.

The present embodiment utilizes a matrix to which one or more amine-containing compounds which inhibit microbial growth in complex media have been bound either covalently or passively. Examples of such matrices include polymers, such as polyethylene or polystyrene; metal, glass and cellulose-based compositions to which the compound has been bound by passive adsorption or by covalent bonding. The coated surface may be placed in contact with a food product, for example, a liquid product such as milk, either as an in-line cartridge or other contact-enhancing system during a transfer step or as a part of the final packaging material. This serves to destroy the contaminating bacteria.

The amine containing compounds of this embodiment are the tertiary amines, partially oxidized tertiary amines, or quaternary ammonium salts, specifically those which have the characteristic of preventing microbial growth in complex media. Representative suitable amine-containing compounds of the former class are those of the formula: 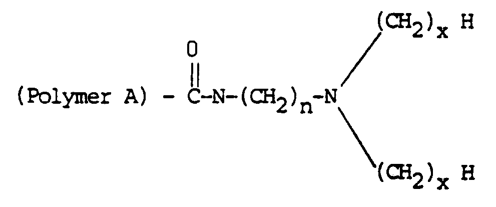
wherein n is from 1 to 6, in particular 1, 2, 3, 4, or 6, and x is from 1 to 18, in particular 1, 2, 4, or 18. The compound of Formula I wherein n is 3 and x is 1 is active against Saccharomyces cerevisiae and the compounds of Formula I wherein n is 2 and x is 1 or wherein n is 3 and x is 2 are active against E. coli.

Representative of the second class are compounds generated by the partial oxidation of tertiary amines conjugated to polystyrene. An example of this are the compounds generated by the treatment of dimethyl-methylamine conjugated to a styrene-divinyl benzene copolymer (polystyrene) with hydrogen peroxide. More generally, the class of compounds that can be rendered effective by treatment with hydrogen peroxide or an equivalent oxidant can be represented by the formula: 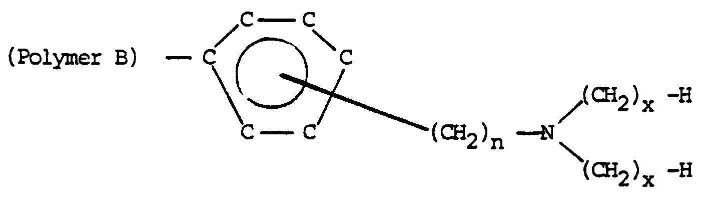
Typically these compounds will have low or no antimicrobial activity in complex media prior to oxidation. Prior to the present invention tertiary amines and quaternary ammonium salts have been reported having antimicrobial activity in buffer solutions or water. However, no amine-containing compounds have been reported prior to this invention which have significant antimicrobial activity in complex media.

An amine-containing compound is useful in accordance with the present embodiment when it prevents growth of bacteria, or fungi in a complex medium in the following test. From 10¹ to 10³ cells/ml of a bacteria are added to pasteurized milk and incubated from 120 to 192 hours at a temperature of 11°C. The amine containing compound is added to the milk in immobilized form on beads, or on the inner surface of the container for the milk, prior to incubation. After incubation and at least 24 hours after adding the amine-containing compound, the milk is tested for the presence of the bacteria by dilution and plate count. A bacteria level below 10⁵ cells/ml is considered acceptable. The test also can be conducted in a fruit juice such as orange juice, apple juice or the like. The micro-organisms used in the test can be bacteria, yeasts or moulds. The amine-containing compound is covalently bound to a polymeric backbone through a linkage such as an amide linkage or a phenylene linkage. Generally the concentration of the immobilized amine-containing compound will be between 2 and 100 µeq/sq.in. Surplus amine-containing compound can be removed by washing after a brief incubation period. The concentration of the immobilized amine-containing compound is sufficient to effect substantial inhibition of the micro-organism from the liquid being treated.

Depending on the micro-organisms present and the parameters of the application, the inhibition process is either selective towards a particular unwanted micro-organism species or non-selective in the sense that most or all contaminating micro-organisms are inhibited. This could be accomplished by inhibition of the bacteria either as an in-line component of a transfer process or as a component of a liquid container itself. Certain solid foods such as fish and meat are degraded by bacterial contamination and the spread of contamination from the surface. It is within the scope of this embodiment to apply the same principle for achievement of increased shelf life of such products by surrounding the solid foods with a liquid and a film or a film alone wherein the amine-containing compound is bound to the film.

Referring to the container 8 shown in the drawing, an amine-containing compound coated on inner surfaces of the container 8 functions to bind and inhibit micro-organisms growing in the liquid.

The embodiment will now be described in greater detail with reference to the following non-limiting examples:

### EXAMPLE (i)

The antimicrobial activity of an amine-containing surface was demonstrated by adding 100 mg of "Amberlite" IRA-68 beads, Lot No. 66F-0148 (supplied by Sigma Chemical Co., St. Louis, Mo., United States of America) to 2 ml of 50 mM Tris buffer (pH 7.5) inoculated with 10⁵ cells/ml of food spoilage micro-organism, such as Pseudomonas fluorescens, Escherichia coli, or Bacillus brevis, in test tubes. These beads are representative of Polyacrylamide surfaces containing N,N- dimethyl aminopropyl groups conjugated to the amide nitrogen of the polymer. Tubes were agitated at 22°C and viable cells remaining were measured by plate count. Within two hours viable cell number, in the presence of the tertiary surface, of Bacillus brevis had decreased more than an order of magnitude and Pseudomonas fluorescens and Escherichia coli viable cell numbers had decreased more than four orders of magnitude.

### EXAMPLE (ii)

The antimicrobial activity of another amine surface was demonstrated by adding 100 mg of "Amberlite" IRA-94 beads, Lot No. 123F-0163 (supplied by the said Sigma Chemical Co.) to 2 ml of 50 mM Tris buffer (pH 7.5) inoculated with 10⁵ cells/ml of Pseudomonas fluorescens, Escherichia coli, or Saccharomyces cerevisiae, in test tubes. The bead surface is representative of surfaces containing 4 meq/ g of partially oxidized dimethyl-methylamine conjugated to a styrene-divinyl benzene copolymer.

Tubes were agitated at 22°C and viable cells remaining were measured by plate count. Within two hours viable cell numbers, in the presence of the tertiary amine surface, had decreased more than four orders of magnitude for all three test organisms.

### EXAMPLE (iii)

A carton containing an actively antimicrobial surface would increase the shelf life of pasteurized fruit juice by inhibiting the growth of the microflora present. To demonstrate this, 100 mg of the dimethyl amino propyl polyacrylamide (IRA-68) beads were added to a test tube containing 2 ml of sterile apple juice. Tubes were inoculated with a Saccharomyces cerevisiae culture to give 10³ cells/ml and incubated at 11°C. The presence of the tertiary amine surface inhibited both the growth rate and total cell number. Organoleptic evaluation of the test samples after seven days revealed that the controls had spoiled while the amine-surface-containing samples had not. This example illustrates the test utilized to identify effective amine-containing compounds in accordance with this embodiment.

### EXAMPLE (iv)

This example shows a tertiary amine surface which is of little effectiveness in inhibiting the growth of Saccharomyces cerevisiae under the conditions described in Example (iii). 100 mg of "Dowex" WGR-2 beads, Lot No. 83F-0120 (supplied by the said Sigma Chemica Co.) were added to 2 ml of apple juice which had been inoculated with S. cerevisiae at 10³ cells/ml and agitated at 11°C. These beads are representative of surfaces containing dimethyl-methylamine conjugated to a styrene- divinyl benzene copolymer. Growth was monitored by plate count; an only slight difference in viable cell count was observed between tubes containing the amine surface and control tubes over a 6 day period. In similar studies, carried out in buffered salt solutions instead of a complex medium, WGR-2 beads were shown to have antimicrobial properties, consistently with the findings of Endo et al., and thus demonstrating that antimicrobial activity in simple media does not correlate with such activity in complex media.

### EXAMPLE (v)

To demonstrate the effectiveness of the dimethyl amino propyl polyacrylamide surface in increasing the shelf life of pasteurized milk, the experiment described in Example (iii) was repeated with non-fat dry milk as the complex medium, "Amberlite" IRA-68 beads as the amine-containing surface, and Pseudomonas fluorescens as the test organism. The presence of the amine surface significantly decreased the growth rate of Pseudomonas fluorescens in this medium.

### EXAMPLE (vi)

This example demonstrates the utility of another amine containing surface for controlling microbial growth in a complex medium. 100 mg of "Amberlite A-21" beads consisting of partially oxidized dimethyl-methylamine conjugated to a styrene-divinyl benzene copolymer were added to 2 ml of sterile apple juice. The partial oxidation of these beads was achieved by prior treatment with hydrogen peroxide. The tubes were inoculated with a Saccharomyces cerevisiae culture to give 10²-10³ cells/ml and incubated at either 6°, 11°, or 25°C. In all cases, growth of the cells was dramatically inhibited by the presence of these surfaces. In addition, spoilage of the juice was found to be greatly retarded relative to the controls.

### EXAMPLE (vii)

This example demonstrates that an immobilized quaternary ammonium compound, known in the prior art as an active antimicrobial surface, is inactive in the presence of a complex medium. A solution of 3-trimethoxysilyl propyloctadecyl dimethyl ammonium chloride (Sylgard -United States Patent 4259103) was prepared by addition to water and this was used to coat the inside of a culture tube. To the tubes was added either a simple buffer solution or sterile apple juice and these media were then inoculated with 10⁵ cell/ml of S. cerevisiae in the case of buffer or 10³ cell/ml in the case of apple juice. The samples were incubated at 11°C. At various timepoints following inoculation samples were withdrawn and viable colony forming units were determined by plate counting. Antifungal activity was demonstrated by a rapid kill (within 8 hours) of the yeast cells in the buffer solution. this observation was in agreement with the prior art. In contrast, however, no decrease in cell viability was observed in the apple juice samples and normal growth was observed over 5 days relative to controls. This demonstrated that the antimicrobial activity of this agent was lost in a complex medium.

### EXAMPLE (viii)

Antifungal activity of the amine-containing surface was demonstrated in a yeast growth medium (Bacto Malt Extract Broth, Difco). 250 mg of "Amberlite" IRA-68 and IRA-94 were put in test tubes, and 5 ml growth medium containing 10³ cells/ml Saccharomyces cerevisiae added to each tube. Growth in tubes with beads and control tube was measured by plate count for 6 days. After 6 days, the number of S. cerevisiae cells in control samples had reached 10⁹ cells/ml, while no growth in either IRA-68 or IRA-94 tubes was detected. The numbers of cells in these tubes were less than 10³ after 6 days incubation.

### EXAMPLE (ix)

Binding of Saccharomyces cerevisiae to tertiary amines was shown by two methods. In the first, columns (90 x 6 mm) were packed with "Amberlite" IRA-68 and IRA-94 beads. The columns were equilibrated with ¼ strength Ringers solution. One ml of S. cerevisiae cells (10⁶ cells/ml) suspended in Ringers solution was applied on the column and eluted with Ringers solution. One ml fractions of eluate were counted in a bacterial counting chamber, and the number of cells eluted was calculated. 45% of the cells could not be eluted in both IRA-68 and IRA94 colums with the Ringers solution. In the second method, designed to directly demonstrate binding in solution, IRA-68 beads were equilibrated in Ringers solution and 250 mg beads put in a 10 ml test tube. 2 ml of S. cerevisiae suspended in Ringers solution was transferred to the tube. The test tube was incubated vertically at room temperature for 2 hours, the pH measured (5.4) and the supernatant decanted off. Sterile Ringers solution was added twice to wash away unbound cells. Binding of S. cerevisiae cells to IRA-68 beads was shown by microscopic examination.

### EXAMPLE (x)

To demonstrate that the inhibitory effect of the amine-containing surfaces on Saccharomyces cerevisiae is directed to the cell membrane, S. cerevisiae cells were suspended in buffer (10⁵ cells/ml) containing 5% sucrose with or without 50 mg/ml "Amberlite" IRA-68. Samples were removed and their plating efficiency on medium containing 0.01% sodium dodecyl sulfate (SDS) determined. SDS is a surfactant and able to disrupt biological membranes. However, the concentration of SDS used (0.01%) is below the lethal concentration for S. cerevisiae. After 70 hours exposure to IRA-68, the cells had an 80% lower plating efficiency on 0.01% SDS medium relative to medium without SDS. Samples which were not exposed to IRA-68 showed no differences in plating efficiency, indicating that exposure to IRA-68 sensitized S. cerevisiae cells to the membrane disrupting effects of SDS at this normally non-lethal concentration.

### EXAMPLE (xi)

Further evidence that exposure to IRA-68 disrupts membrane integrity of Saccharomyces cerevisiae was obtained by staining cells with Trypan Blue. Trypan Blue staining indicates viability of eukaryotic cells by a dye exclusion mechanism. Cells which have an intact cell membrane do not allow the dye to enter the cell and therefore do not stain. Non-viable or damaged cells, having a permeable membrane, allow the dye to enter and stain blue. S. cerevisiae cells were suspended in buffer (10⁵ cells/ml) containing 5% sucrose with or without 50 mg/ml IRA-68. After 120 hrs. exposure to IRA-68, greater than 80% of the S. cerevisiae cells stained blue while only 2-3% of control cells, which were not exposed to IRA-68, stained.

### EMBODIMENT III

The present embodiment will be described herein with specific reference to binding iron from foods such as milk and fruit-based drinks, including fruit drinks and nectars. However, it is to be understood that the present embodiment is useful for binding iron from liquid phases in general.

Preferably a matrix is utilized to which an iron binding composition has been bound either covalently or non-covalently. Examples of such matrices include polymers, such as polyethylene or polystyrene; glass and cellulose based compositions to which the iron binding composition has been bound by passive adsorption or by covalent bonding. The surface coated with the iron binding composition can be placed in contact with a food product, for example a liquid product such as fruit juice, either as an in-line cartridge or another contact-enhancing system during a transfer step or as a part of the packaging material. This serves to remove the iron which supports micro-organisms growth by chelation to the solid phase, with the ultimate result of prolonging the shelf life of the packaged product and/or reducing or preventing growth of the undesirable or pathogenic organisms.

Good contact between the liquid product in a container 8 and the iron binding composition bound to the matrix in the container is provided by relative motion between the liquid 22 and the binding surface in the container. Alternatively, the iron binding composition may be bound to the surface of a separate device which moves and agitates the liquid product. Separation of the bound iron is obtained when the device is removed or the liquid material is poured out of the container, the iron remaining bonded to the inner surface of the emptied container.

Suitable iron binding compositions include deferriferrichrome A, dipyridyl, pyrimine or mixtures thereof.

The nature of the immobilization of the iron binding composition to a solid support can be either covalent or non-covalent. The important characteristic of this interaction is that the composition which is immobilized to the support should remain tightly bound and not be released during the step of iron binding. Covalent binding of the iron binding composition can be accomplished by a wide variety of techniques which are well known in the art. In some cases, it may be desirable to include linkers or spacer arms to allow the iron binding composition some additional freedom of movement and/or exposure to the liquid material. These can be incorporated during the immobilization step.

As an alternative to covalent immobilization it may be desirable under some circumstances to bind the iron binding composition non-covalently to a material contact surface by hydrophobic interaction. An aqueous solution of an iron binding composition is incubated in contact with the material contact surface to which the iron binding composition is to be bound.

Iron binding compositions bound to material contact surfaces in this fashion are stable and the prepared substrates can be stored either in a buffer solution or dried without loss of activity. The concentration of the immobilized iron binding composition is sufficient to effect substantial inhibition of the micro-organisms in liquid or solid material treated.

When iron-containing media are placed in contact with an insoluble matrix comprising the iron binding composition linked to a substrate, the iron can be quantitatively transferred from a free to a bound form where it is no longer available for microbial growth. This quantitative transfer occurs as long as there is excess binding capacity of the insoluble matrix over the free iron and as long as the iron is not complexed in an inaccessible form such as heme.

The result of this transfer of iron to an insoluble form is that the concentration of free iron is reduced significantly below the lower limit required for microbial growth. Thus, the growth of micro-organisms contaminating the medium in contact with the iron binding matrix is lowered relative to the situation in which the medium is not placed in contact with such a surface, with the shelf life being correspondingly increased. Under these conditions, the only ways in which micro-organisms can overcome the inhibition is by degrading the iron binding matrix or by elaborating iron binding agents (siderophores) which have higher binding constants than the matrix. In these cases a substantial lag will still be observed before growth commences. This is especially true in the latter case because of the kinetic stability of the insoluble complexes.

Referring to the container 8 shown in the drawing, an iron binding composition bound to inner surfaces of the container 8 functions to bind and immobilize the iron in the liquid food 22. If desired, a binding protein and/or a substance which blocks the metabolism of the micro-organism also can be bound to some or all of the inner surfaces of the container 8.

The following examples again illustrate the present embodiment.

### EXAMPLE I

This example illustrates the immobilization of an iron binding composition on glass beads.

Ferrichrome A was purified directly from the medium of Ustilago spherogena by recrystallization.

This iron chelator was covalently bound to glass in the iron saturated form which protects the iron binding domain.

40 mg of the recrystallized ferrichrome A was dissolved in 40 ml acetone containing 0.4 ml pyridine. To this was then added 40 mg of tosyl chloride and the reaction was allowed to stir at room temperature for one hour. At this point, 2 grams of aminopropyl controlled pore glass beads was added to the mixture and the reaction was allowed to continue for an additional 4 hours. The red coloured ferrichrome A was then seen to be largely associated with the glass beads. The reaction mixture was filtered and washed twice with acetone, twice with water and finally, two more times with acetone. The orange-red beads were allowed to dry.

### EXAMPLE II

In order to evaluate the immobilized ferrichrome A product produced by the procedure of Example I, non-specific background for reactivity with unbound iron was eliminated by the process of this example.
Unreacted primary amino groups on the ferrichrome A-glass beads were blocked by reaction with acetic anhydride. Two grams of ferrichrome A-glass beads were suspended in 10 ml of 100:1 acetone/pyridine. To this was added 2.73 ml of acetic anhydride and the reaction was incubated for at least one hour. The beads were washed in acetone, followed by two washes with water and an additional two washes with acetone. The derivatized beads were dried in vacuo.

### EXAMPLE III

This example illustrates a method for regenerating an iron binding composition containing bound iron.

Bound iron was removed from the immobilized ferrichrome A by suspending 1 gram of the ferrichrome A-glass beads in 5 ml of water and adding 0.25 ml of 1 M KSCN and 5 mg of sodium dithionite. The reaction mixture was allowed to stand for at least one hour during which time the colour was observed to be lost from the glass beads. The deferrated (deferriferrichrome A) beads were then washed twice with water and twice with acetone. The deferriferrichrome A-glass beads were then dried and sterilized by treatment with 80% ethanol.

### EXAMPLE IV

This example illustrates growth inhibition of bacteria by the method of this embodiment.

A psychrotrophic strain of Pseudomonas fluorescens was isolated from spoiled milk. 10³ CFU of these cells were inoculated into 5 ml aliquots of a medium consisting of 6 grams of Na₂HP0₄, 3 grams of KH₂P0₄, 0.5 grams of NaCl, 1 gram of NH₄Cl, 2 grams of glucose, 0.002 M MgSO₄, and 0.0001 M CaCl₂. The iron concentration of the medium was determined to be 1.2 µmole/l. At the same time as the cells were added, 10 mg of the DFFA derivatized glass beads were added to some of the samples while acetic anhydride capped aminopropyl glass beads were added to the others. The samples were incubated at 11°C and growth was evaluated by determining CFU/ml by plate counting at various times. The results showed that the growth of the bacterial cells was significantly inhibited by the addition of the immobilized iron chelator. Growth eventually occurred in these samples after an extended lag phase.

### EXAMPLE V

This example illustrates that desferrioxamine B is not suitable as an iron binding composition in the present method.

Desferrioxamine B (Desferal™, Ciba-Geigy) was coupled to agarose beads which had been modified with CNBr as is known in the art. Covalent binding occurred between the primary amino group of the Desferal and the agarose beads and the iron chelator retained its iron binding ability as determined by studies analogous to those described in previous examples. A growth study similar to that detailed in Example IV was carried out except that an equivalent amount of Desferal-agarose was added instead of DFFA-glass beads. No growth inhibition was observed even when a substantial excess of iron binding matrix was added over the available iron; thus, the bacteria were able to obtain iron bound to the Desferal-agarose either by direct or indirect competition.

### EXAMPLE VI

(This Example could also be considered as inserted in EMBODIMENT II).

This example illustrates the denial of components necessary for growth of Saccharomyces by tertiary amines in a growth medium.

IRA-94 and IRA-68 (Rohm & Haas) were incubated with a complex medium containing several pools of growth factors, the medium containing the following constituents:-

| Nitrogen Source | |
|---|---|
| Ammonium Sulphate | 3.5 g/l |

| Carbon Source | |
|---|---|
| Sucrose | 5.0 g/l |

| Vitamins | | |
|---|---|---|
| Pool 1 | (Biotin | 2.0 µg/l |
| | (Calcium Pantothenate | 400.0 µg/l |
| | (Folic Acid | 2.0 µg/l |
| Pool 2 | (Inositol | 2000.0 µg/l |
| | (Niacin | 400.0 µg/l |
| | (p-Aminobenzoic Acid | 200.0 µg/l |
| Pool 3 | (Pyridoxine HCL | 400.0 µg/l |
| | (Riboflavin | 200.0 µg/l |
| | (Thiamine HCL | 400.0 µg/l |

| Trace Elements | | |
|---|---|---|
| Pool 4 | (Boric Acid | 500.0 µg/l |
| | (Copper Sulphate | 40.0 µg/l |
| | (Potassium Iodide | 100.0 µg/l |
| Pool 5 | (Manganese Sulphate | 400.0 µg/l |
| | (Sodium Molybdate | 200.0 µg/l |
| | (Zinc Sulphate | 400.0 µg/l |
| | (Iron Chloride | 200.0 µg/l |

| Salts | |
|---|---|
| Potassium Phosphate Monobasic | 1.0 g/l |
| Magnesium Sulphate | 0.5 g/l |
| Sodium Chloride | 0.1 g/l |
| Calcium Chloride | 0.1 g/l |

After 48 hours of exposure of the medium to the IRA68 or IRA94, the treated medium was inoculated with Saccharomyces cells. Medium treated with IRA-94 beads did not support growth while medium treated with IRA-68 beads showed growth comparable to the non-treated medium. The medium treated with IRA-94 did support growth of Saccharomyces when all pools of nutrients where added back at the same time.

The present method has the following advantages:-
A) The active substances are easily bonded to the matrix, either passively or actively (covalently) by chemical treatment.
B) The matrix can be polymer, glass, stainless steel, etc.
C) Only non-toxic, food-compatible materials are used.
D) Migration of the active substances and the matrix materials to the material being treated is negligible.
E) The organoleptic and nutritional qualities of food are maintained.
F) Active, affinity-based extraction systems of cells or growth factors are achieved, which leave other constituents intact and unaffected.
G) The systems are biologically based.
H) Specific and non-specific affinities are available as in Embodiments I and II.
I) Regeneration of active substances is available for Embodiments I and III.
J) Active microbiological control systems are provided for microbiological stabilization after filling of containers.

### INDUSTRIAL APPLICABILITY

The present embodiments are applicable in a variety of industrial fields, particularly in the packaging field.

## Claims

1. A method for controlling the growth of micro-organisms in a packaged material (22) in the form of a complex medium and comprising of liquid, wherein the material (22) is contacted with a substantially insoluble, immobilized, growth-inhibiting substance at a surface (10, 12, 14, 16) of a packaging material (8), said substance being of a composition to be active upon said packaged material (22), characterized in that the at least one active substance is not an enzyme and in that said activity is promoted by producing relative motion between the material and the active substance(s).

2. A method according to claim 1, wherein the or each active substance is selected from the group consisting of lectins, polysaccharides, antibodies, metal chelators, and amine-containing compounds.

3. A method according to claim 1 or 2, wherein said active substance(s) comprise(s) a binding substance having a binding affinity to particular constituents in said packaged material (22), thereby to bind said particular constituents, said binding substance having a high binding affinity to said particular constituents, but a low binding affinity to other constituents in said packaged material (22) which tend to inhibit the binding of said particular constituents.

4. A method according to claim 3, wherein said particular constituents are said micro-organisms.

5. A method according to claim 2, 3 or 4, wherein said active substance(s) comprise(s) an amine-containing compound, the amine being available for and capable of inflicting sufficient damage upon said micro-organisms in said material (22) to inhibit their growth in said material (22).

6. A method according to claim 5, wherein said compound is a tertiary amine.

7. A method according to claim 5, wherein said compound is a partially oxidized tertiary amine.

8. A method comprising providing a material (22) in the form of a complex medium which may contain micro-organisms, and controlling the growth of said micro-organisms in said complex medium (22) by contacting the complex medium with a surface comprised of an active substance in the form of a binding substance having a binding affinity to said micro-organisms in said complex medium, thereby to bind said micro-organisms, said substance having a high binding affinity for said micro-organisms, but a low binding affinity for constituents in said complex medium other than said micro-organisms, said active substance not being an enzyme.

9. A method according to claim 8, wherein said binding substance is a lectin or an amine-containing compound.

10. A method for controlling the growth of micro-organisms in a material (22) in the form of a complex medium, wherein the material (22) is contacted with an active substance, characterized in that the active substance is in the form of an immobilized amine-containing compound, the amine being available for and capable of inflicting sufficient damage upon micro-organisms in said material (22) to inhibit their growth in said material (22).

11. A method according to claim 10, wherein said compound is a tertiary amine.

12. A method according to claim 10, wherein said compound is a partially oxidized tertiary amine.

13. A method according to claim 11, wherein said compound is of the formula: 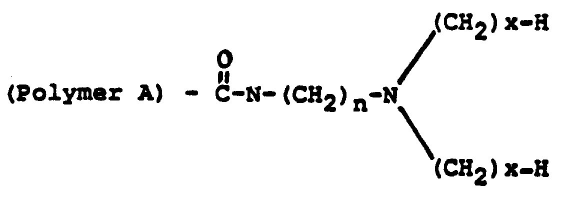 wherein n is from 1 to 6 and x is from 1 to 18.

14. A method according to claim 12, wherein said tertiary amine prior to partial oxidation is of the formula: 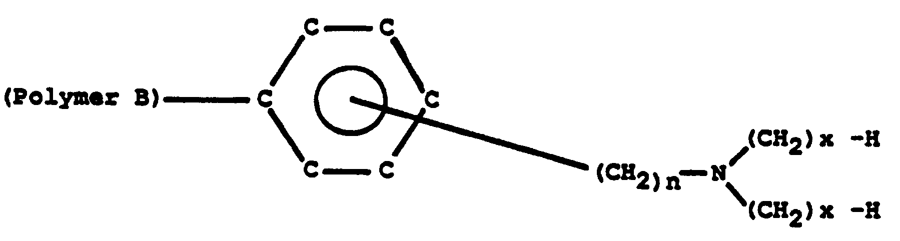 wherein n is from 1 to 6 and x is from 1 to 18.

15. A method for controlling the growth of micro-organisms in a material (22) in the form of a complex medium by contacting the complex medium with an active substance in the form of a binding substance comprising a binding composition having affinity for at least one trace metal constituting a biological growth factor of micro-organisms in said complex medium, wherein there is used a binding composition providing a binding constant and a kinetic stability for binding said trace metal(s) sufficient for the binding composition successfully to compete with micro-organisms for said trace metal(s) and to retain the bound trace metal(s), thus restricting the availability of said trace metal(s) to said micro-organisms.

16. A method according to claim 15, wherein the binding composition is pyrimine.

17. A method according to claim 15, wherein said binding composition is an amine-containing compound.

18. A method according to claim 15, wherein the trace metal is ferric iron and the binding composition has a binding constant for ferric iron of at least 10³⁰ litres/mole and a capacity of forming a kinetically stable complex with ferric iron at least 10 times more stable than that of ferrioxamine B.

19. A method according to claim 3 or any one of claims 4 to 7 as appended to claim 3, wherein said packaging material (8) is a distribution container (8), the binding action being enhanced by relative motion between the material (22) and said binding substance due to handling of the container (8).

20. A method according to claim 8, 10 or 15, wherein the material (22), which is fluid, is contacted with said active substance by being passed over a surface at which is said active substance.

21. A method according to claim 8, 10 or 15, wherein the activity of said active substance is enhanced by relative motion between the material (22) and said active substance.

22. Packaging material (8) having substantially insoluble, immobilized, growth-inhibiting substance at a surface (10, 12, 14, 16) thereof for contacting another material (22) in the form of a complex medium and comprising liquid, said substance being of a composition to be active upon said other material (22) to inhibit the growth of micro-organisms in said other material (22), characterized in that there is a plurality of active substances which are not enzymes.

23. Packaging material according to claim 22, wherein the active substances are selected from the group consisting of lectins, polysaccharides, antibodies, metal chelators, and amino-containing compounds.

24. Packaging material according to claim 21 or 22, wherein said active substances comprise a binding substance having a binding affinity to particular constituents in said other material (22), thereby to bind said particular constituents, said binding substance having a high binding affinity to said particular constituents and a low binding affinity to other constituents in said other material (22) which tend to inhibit the binding of said particular constituents.

25. Packaging material according to claim 24, said particular constituents being said micro-organisms.

26. Packaging material according to claim 24 or 25, wherein said binding substance is a lectin or an amine-containing compound.

27. Packaging material according to any one of claims 22 to 26, wherein an enzyme is associated with said active substances.

28. Packaging material (8) having substantially insoluble, immobilized, growth-inhibiting substance at a surface (10, 12, 14, 16) thereof for contacting another material (22) in the form of a complex medium and comprising liquid, said substance being of a composition to be active upon said other material (22) to inhibit the growth of micro-organisms in said other material (22), wherein said active substance(s) comprise(s) an amine-containing compound of which the amine is available for and capable of inflicting sufficient damage upon said micro-organisms in said other material (22) to inhibit their growth in said other material (22).

29. Packaging material according to claim 28, wherein said compound is a tertiary amine.

30. Packaging material (8) having substantially insoluble, immobilized, growth-inhibiting substance at a surface (10, 12, 14, 16) thereof for contacting another material (22) in the form of a complex medium and comprising liquid, said substance being of a composition to be active upon said other material (22) to inhibit the growth of micro-organisms in said other material (22), wherein said active substance(s) comprise(s) a binding substance in the form of a binding composition having affinity for at least one trace metal constituting a biological growth factor and providing a binding constant and a kinetic stability for binding said trace metal(s) sufficient for the binding composition successfully to compete for said trace metal(s) with said micro-organisms and to retain the bound trace metal(s) thus restricting the availability of said growth factor(s) to said micro-organisms.

31. Packaging material according to claim 30, wherein the binding composition is pyrimine.

32. Packaging material according to claim 30, wherein the binding composition has affinity for ferric iron and a binding constant for ferric iron of at least 10³⁰ litres/mole and a capacity of forming a kinetically stable complex with ferric iron at least 10 times more stable than that of ferrioxamine B.

33. Packaging material according to any one of claims 22 to 27, wherein said active substances are at different zones of said surface (10, 12, 14, 16).

34. Packaging material according to any one of claims 22 to 27, wherein said active substances are intermixed over said surface (10, 12, 14, 16).

35. In combination, a complex medium (22), a binding substance having a binding affinity to micro-organisms in said medium for thereby binding said micro-organisms, and a biocidal agent for inactivating said micro-organisms, said binding substance being immobilized at a surface contacting said complex medium (22).

36. A combination according to claim 35, wherein said binding substance is a lectin or an amine-containing compound.

37. A combination according to claim 36, wherein said compound is a tertiary amine.

38. A combination according to any one of claims 35 to 37, wherein said biocidal agent is an amine-containing compound.

39. Use of an active substance for inflicting sufficient damage upon micro-organisms in a complex medium to inhibit their growth in said medium, characterised in that said active substance comprises an amine-containing compound immobilized at a surface contacting said complex medium.

40. Use of an active substance according to claim 39, wherein said compound is a tertiary amine.

41. Use of an active substance according to claim 39, wherein said compound is a partially oxidized tertiary amine.

## Patentansprüche

1. Verfahren zum Steuern des Wachstums von Mikroorganismen in einem verpackten Material (22) in Form eines komplexen Mediums und bestehend aus Flüssigkeit, worin das Material (22) mit einer im wesentlichen unlöslichen, immobilisierten, wachstumshemmenden Substanz an einer Oberfläche (10, 12, 14, 16) eines Verpackungsmaterials (8) in Berührung gebracht wird, wobei die Substanz eine Zusammensetzung aufweist, die hinsichtlich des verpackten Materials (22) aktiv sein soll, dadurch gekennzeichnet, daß die mindestens eine aktive Substanz kein Enzym ist und daß die Aktivität durch das Erzeugen von relativer Bewegung zwischen dem Material und der(den) aktiven Substanz(en) gefördert wird.

2. Verfahren nach Anspruch 1, worin die oder jede aktive Substanz aus der Gruppe ausgewählt wird, die sich aus Lektinen, Polysacchariden, Antikörpern, Metallchelatoren und Amin enthaltenden Verbindungen zusammensetzt.

3. Verfahren nach Anspruch 1 oder 2, worin die aktive(n) Substanz(en) eine Bindesubstanz umfaßt(umfassen), welche eine Bindungsaffinität zu bestimmten Bestandteilen im verpackten Material (22) aufweist, um dadurch die bestimmten Bestandteile zu binden, wobei die Bindesubstanz eine große Bindungsaffinität zu den bestimmten Bestandteilen jedoch eine geringe Bindungsaffinität zu anderen Bestandteilen im verpackten Material (22) aufweist, welche dazu neigen, die Bindung der bestimmten Bestandteile zu hemmen.

4. Verfahren nach Anspruch 3, worin die bestimmten Bestandteile die Mikroorganismen sind.

5. Verfahren nach Anspruch 2, 3 oder 4, worin die aktive(n) Substanz(en) eine Amin enthaltende Verbindung umfaßt(umfassen), wobei das Amin dazu verfügbar und in der Lage ist, Mikroorganismen im Material (22) in ausreichendem Maße zu schädigen, um ihr Wachstum im Material (22) zu hemmen.

6. Verfahren nach Anspruch 5, worin die Verbindung ein tertiäres Amin ist.

7. Verfahren nach Anspruch 5, worin die Verbindung ein teilweise oxidiertes, tertiäres Amin ist.

8. Verfahren, welches das Bereitstellen eines Materials (22) in Form eines komplexen Mediums, welches Mikroorganismen enthalten kann, und das Steuern des Wachstums der Mikroorganismen im komplexen Medium (22) durch Zusammenbringen des komplexen Mediums mit einer Oberfläche umfaßt, die eine aktive Substanz in Form einer Bindesubstanz aufweist, welche eine Bindungsaffinität zu den Mikroorganismen im komplexen Medium aufweist um dadurch die Mikroorganismen zu binden, wobei die Substanz eine hohe Bindungsaffinität für die Mikroorganismen jedoch eine geringe Bindungsaffinität für jene Bestandteile im komplexen Medium aufweist, die nicht die Mikroorganismen sind, wobei die aktive Substanz kein Enzym ist.

9. Verfahren nach Anspruch 8, worin die Bindesubstanz ein Lektin oder eine Amin enthaltende Verbindung ist.

10. Verfahren zum Steuern da Wachstums von Mikroorganismen in einem Material (22) in Form eines komplexen Mediums, worin das Material (22) mit einer aktiven Substanz in Berührung gebracht wird, dadurch gekennzeichnet, daß die aktive Substanz in Form einer immobilisierten, Amin enthaltenden Verbindung vorliegt, wobei das Amin dazu verfügbar und in der Lage ist, Mikroorganismen im Material (22) in ausreichendem Maße zu schädigen, um ihr Wachstum im Material (22) zu hemmen.

11. Verfahren nach Anspruch 10, worin die Verbindung ein tertiäres Amin ist.

12. Verfahren nach Anspruch 10, worin die Verbindung ein teilweise oxidiertes, tertiäres Amin ist.

13. Verfahren nach Anspruch 11, worin die Verbindung die folgende Formel aufweist: 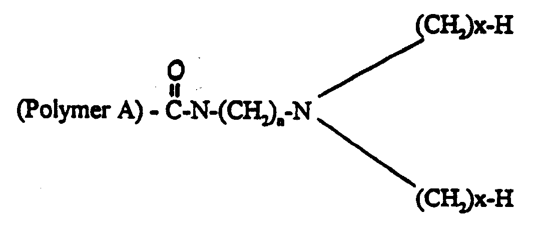 worin n von 1 bis 6 und x von 1 bis 18 beträgt.

14. Verfahren nach Anspruch 12, worin das tertiäre Amin vor der teilweisen Oxidation die folgende Formel aufweist: 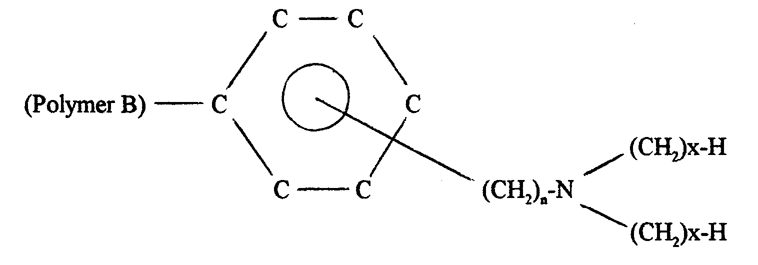 worin n von 1 bis 6 und x von 1 bis 18 beträgt.

15. Verfahren zum Steuern des Wachstums von Mikroorganismen in einem Material (22) in Form eines komplexen Mediums durch Zusammenbringen des komplexen Mediums mit einer aktiven Substanz in Form einer Bindesubstanz, die eine Bindezusammensetzung umfaßt, welche eine Affinität für zumindest ein Spurenmetall aufweist, das einen biologischen Wachstumsfaktor von Mikroorganismen im komplexen Medium darstellt, worin eine Bindezusammensetzung verwendet wird, welche eine Bindungskonstante und eine kinetische Stabilität zum Binden des(der) Spurenmetalls(Spurenmetalle) schafft, welche ausreicht, daß die Bindezusammensetzung erfolgreich mit Mikroorganismen in Konkurrenz hinsichtlich des(der) Spurenmetalls(Spurenmetalle) tritt und das(die) gebundene(n) Spurenmetall(e) zurückhält, wodurch die Verfügbarkeit des(der) Spurenmetalls(Spurenmetalle) für die Mikroorganismen eingeschränkt wird.

16. Verfahren nach Anspruch 15, worin die Bindezusammensetzung Pyrimin ist.

17. Verfahren nach Anspruch 15, worin die Bindezusammensetzung eine Amin enthaltende Verbindung ist.

18. Verfahren nach Anspruch 15, worin das Spurenmetall ein dreiwertiges Eisen ist und die Bindezusammensetzung eine Bindungskonstante für dreiwertiges Eisen von mindestens 10³⁰ Liter/Mol und eine Kapazität zum Bilden eines kinetisch stabilen Komplexes mit dreiwertigem Eisen aufweist, der mindestens zehnmal stabiler ist als jener von Ferrioxamin B.

19. Verfahren nach Anspruch 3 oder einem der Ansprüche 4 bis 7, die auf Anspruch 3 rückbezogen sind, worin das Verpackungsmaterial (8) ein Verteilungsbehälter (8) ist, wobei die Bindewirkung durch die durch die Handhabung des Behälters (8) bewirkte relative Bewegung zwischen dem Material (22) und der Bindesubstanz verstärkt wird.

20. Verfahren nach Anspruch 8, 10 oder 15, worin das Material (22), welches flüssig ist, mit der aktiven Substanz in Berührung gebracht wird, indem es über eine Oberfläche hinwegführt wird, auf welcher sich die aktive Substanz befindet.

21. Verfahren nach Anspruch 8, 10 oder 15, worin die Aktivität der aktiven Substanz durch die relative Bewegung zwischen dem Material (22) und der aktiven Substanz verstärkt wird.

22. Verpackungsmaterial (8), welches an einer seiner Oberflächen (10, 12, 14, 16) eine im wesentlichen unlösliche, immobilisierte, wachstumshemmende Substanz aufweist, für das Zusammenbringen mit einem anderen Material (22) in Form eines komplexen Mediums, welches Flüssigkeit umfaßt, wobei die Substanz eine Zusammensetzung aufweist, die hinsichtlich des anderen Materials (22) aktiv wird, um das Wachstum von Mikroorganismen im anderen Material (22) zu hemmen, dadurch gekennzeichnet, daß eine Vielzahl an aktiven Substanzen vorliegt, welche keine Enzyme sind.

23. Verpackungsmaterial nach Anspruch 22, worin die aktiven Substanzen aus der Gruppe ausgewählt sind, welche sich aus Lektinen, Polysacchariden, Antikörpern, Metallchelatoren und Amin enthaltenden Verbindungen zusammensetzt.

24. Verpackungsmaterial nach Anspruch 21 oder 22, worin die aktiven Substanzen eine Bindesubstanz umfassen, welche eine Bindungsaffinität zu bestimmten Bestandteilen im anderen Material (22) aufweist, um dadurch die bestimmten Bestandteile zu binden, wobei die Bindesubstanz eine große Bindungsaffinität zu den bestimmten Bestandteilen und eine geringe Bindungsaffinität zu anderen Bestandteilen im anderen Material (22) aufweist, welche dazu neigen, die Bindung der bestimmten Bestandteile zu hemmen.

25. Verpackungsmaterial nach Anspruch 24, wobei die bestimmten Bestandteile die Mikroorganismen sind.

26. Verpackungsmaterial nach Anspruch 24 oder 25, worin die Bindesubstanz ein Lektin oder eine Amin enthaltende Verbindung ist.

27. Verpackungsmaterial nach einem der Ansprüche 22 bis 26, worin ein Enzym mit den aktiven Substanzen assoziiert ist.

28. Verpackungsmaterial (8) welches an einer seiner Oberflächen (10, 12, 14, 16) eine im wesentlichen unlösliche, immobilisierte, wachstumshemmende Substanz aufweist, für das Zusammenbringen mit einem anderen Material (22) in Form eines komplexen Mediums, welches Flüssigkeit umfaßt, wobei die Substanz eine Zusammensetzung aufweist, die hinsichtlich des anderen Materials (22) aktiv wird, um das Wachstum von Mikroorganismen im anderen Material (22) zu hemmen, worin die aktive(n) Substanz(en) eine Amin enthaltende Verbindung umfaßt (umfassen), wobei das Amin dazu verfügbar und in der Lage ist, Mikroorganismen im anderen Material (22) in ausreichendem Maße zu schädigen, um ihr Wachstum im anderen Material (22) zu hemmen.

29. Verpackungsmaterial nach Anspruch 28, worin die Verbindung ein tertiäres Amin ist.

30. Verpackungsmaterial (8), welches an einer seiner Oberflächen (10, 12, 14, 16) eine im wesentlichen unlösliche, immobilisierte, wachstumshemmende Substanz aufweist, für das Zusammenbringen mit einem anderen Material (22) in Form eines komplexen Mediums, welches Flüssigkeit umfaßt, wobei die Substanz eine Zusammensetzung aufweist, die hinsichtlich des anderen Materials (22) aktiv wird, um das Wachstum von Mikroorganismen im anderen Material (22) zu hemmen, worin die aktive(n) Substanz(en) eine Bindesubstanz in Form einer Bindezusammensetzung umfaßt(umfassen), welche eine Affitität für zumindest ein Spurenmetall aufweist, welches einen biologischen Wachstumsfaktor darstellt, und eine Bindungskonstante und eine kinetische Stabilität zum Binden des(der) Spurenmetalls(Spurenmetalle) schafft, die ausreicht, daß die Bindezusammensetzung erfolgreich mit Mikroorganismen in Konkurrenz hinsichtlich des(der) Spurenmetalls(Spurenmetalle) tritt und das(die) gebundene(n) Spurenmetall(e) zurückhält, wodurch die Verfügbarkeit des(der) Wachstumsfaktors(Wachstumsfaktoren) für die Mikroorganismen eingeschränkt wird.

31. Verpackungsmaterial nach Anspruch 30, worin die Bindezusammensetzung Pyrimin ist.

32. Verpackungsmaterial nach Anspruch 30, worin die Bindezusammensetzung eine Affinität für dreiwertiges Eisen und eine Bindungskonstante für dreiwertiges Eisen von mindestens 10³⁰ Liter/Mol und eine Kapazität zum Bilden eines kinetisch stabilen Komplexes mit dreiwertigem Eisen aufweist, der mindestens zehnmal stabiler ist als jener von Ferrioxamin B.

33. Verpackungsmaterial nach einem der Ansprüche 22 bis 27, worin die aktiven Substanzen sich in unterschiedlichen Zonen der Oberfläche (10, 12, 14, 16) befinden.

34. Verpackungsmaterial nach einem der Ansprüche 22 bis 27, worin die aktiven Substanzen über die Oberfläche (10, 12, 14, 16) hinweg untereinander vermischt werden.

35. Kombination eines komplexen Mediums (22), einer Bindesubstanz mit einer Bindungsaffinität zu Mikroorganismen im Medium, um dadurch die Mikroorganismen zu binden und eines Biozids zum Inaktivieren der Mikroorganismen, wobei die Bindesubstanz an einer Oberfläche, welche mit dem komplexen Medium (22) in Berührung kommt, immobilisiert ist.

36. Kombination nach Anspruch 35, worin die Bindesubstanz ein Lektin oder eine Anin enthaltende Verbindung ist.

37. Kombination nach Anspruch 36, worin die Verbindung ein tertiäres Amin ist.

38. Kombination nach einem der Ansprüche 35 bis 37, worin das Biozid eine Amin enthaltende Verbindung ist.

39. Verwendung einer aktiven Substanz, um Mikroorganismen in einem komplexen Medium in ausreichendem Maße zu schädigen, um ihr Wachstum im Medium zu hemmen, dadurch gekennzeichnet, daß die aktive Substanz eine Amin enthaltende Verbindung umfaßt, welche an einer Oberfläche, die mit dem komplexen Medium in Berührung kommt, immobilisiert ist.

40. Verwendung einer aktiven Substanz nach Anspruch 39, worin die Verbindung ein tertiäres Amin ist.

41. Verwendung einer aktiven Substanz nach Anspruch 39, worin die Verbindung ein teilweise oxidiertes, tertiäres Amin ist.

## Revendications

1. Méthode de contrôle de la croissance de micro-organismes dans un matériau emballé (22) se présentant comme un milieu complexe comprenant un liquide, dans laquelle le matériau (22) est en contact avec une substance essentiellement insoluble, immobilisée, inhibitrice de croissance, disposée à la surface (10, 12, 14, 16) d'un matériau d'emballage (8), ladite substance ayant une composition permettant de réagir en présence dudit matériau emballé (22), caractérisée en ce qu'une substance active au moins n'est pas une enzyme, et en ce que ladite réaction est favorisée en produisant un mouvement relatif entre le matériau et la - ou les - substance(s) active(s).

2. Méthode selon la revendication 1, dans laquelle la - ou chacune des - substance(s) active(s) est choisie parmi le groupe constitué par les lectines, polysaccharides, anticorps, chélateurs métalliques, et composés à base d'amine.

3. Méthode selon les revendications 1 ou 2, dans laquelle ladite substance active comprend - ou lesdites substances actives comprennent - un liant ayant une affinité de liaison avec des constituants spécifiques dudit matériau emballé (22), afin de lier lesdits constituants spécifiques, ledit liant ayant une grande affinité de liaison avec lesdits constituants spécifiques, mais une faible affinité de liaison avec les autres constituants dudit matériau emballé (22) qui tendent à inhiber la liaison avec lesdits constituants spécifiques.

4. Méthode selon la revendication 3, dans laquelle lesdits constituants spécifiques sont lesdits micro-organismes.

5. Méthode selon les revendications 2, 3 ou 4, dans laquelle ladite substance active comprend - ou lesdites substances actives comprennent - un composé à base d'amine, l'amine étant prévue pour - et capable de - provoquer une destruction des micro-organismes dudit matériau (22) suffisante pour inhiber leur croissance dans ledit matériau (22).

6. Méthode selon la revendication 5, dans laquelle ledit composé est une amine tertiaire.

7. Méthode selon la revendication 5, dans laquelle ledit composé est une amine tertiaire partiellement oxydée.

8. Méthode comprenant la fourniture d'un matériau (22) se présentant sous la forme d'un milieu complexe susceptible de contenir des micro-organismes, et le contrôle de la croissance desdits micro-organismes dans ledit milieu complexe (22), en mettant en contact le milieu complexe avec une surface comprenant une substance active se présentant comme un liant ayant une affinité de liaison avec lesdits micro-organismes dudit milieu complexe, pour se lier auxdits micro-organismes, ladite substance ayant une grande affinité de liaison avec lesdits micro-organismes, mais une faible affinité de liaison avec les constituants dudit milieu complexe autres que lesdits micro-organismes, ladite substance active n'étant pas une enzyme.

9. Méthode selon la revendication 8, dans laquelle ledit liant est une lectine ou un composé à base d'amine.

10. Méthode de croissance des micro-organismes dans un matériau (22) se présentant comme un milieu complexe, dans laquelle le matériau (22) est mis en contact avec une substance active, caractérisée en ce que la substance active se présente comme un composé immobilisé à base d'amine, l'amine étant prévue pour - et capable de - provoquer une destruction des micro-organismes dudit matériau (22) suffisante pour inhiber leur croissance dans ledit matériau (22).

11. Méthode selon la revendication 10, dans laquelle ledit composé est une amine tertiaire.

12. Méthode selon la revendication 10, dans laquelle ledit composé est une amine tertiaire partiellement oxydée.

13. Méthode selon la revendication 11, dans laquelle ledit composé a la formule suivante : 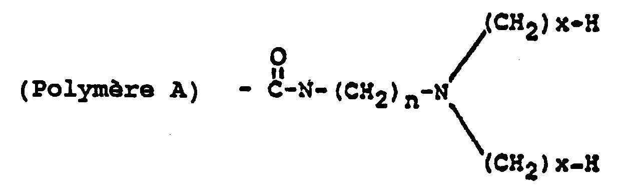 dans laquelle n est compris entre 1 et 6, et x est compris entre 1 à 18.

14. Méthode selon la revendication 12, dans laquelle ladite amine tertiaire, avant son oxydation partielle, a la formule suivante : 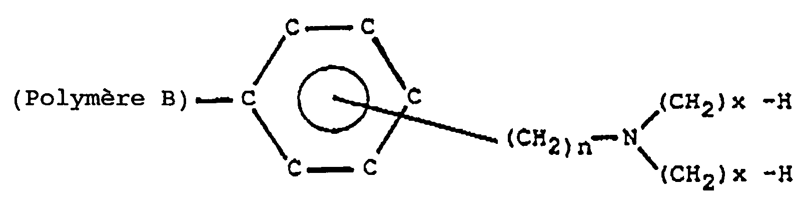 dans laquelle n est compris entre 1 et 6, et x est compris entre 1 à 18.

15. Méthode de contrôle de la croissance des micro-organismes dans un matériau (22) se présentant comme un milieu complexe, en mettant en contact le milieu complexe avec une substance active se présentant comme un liant comprenant un composé de liaison ayant une affinité pour au moins un métal de trace constituant un facteur de croissance biologique de micro-organismes dans ledit milieu complexe, dans laquelle il est utilisé un composé de liaison donnant une constante de liaison et une stabilité cinétique pour lier le métal - ou les métaux - de trace, qui soient suffisantes pour que le composé de liaison réussisse à concurrencer les micro-organismes vis-à-vis dudit métal - ou desdits métaux - de trace, et pour retenir le métal - ou les métaux - de trace lié(s), restreignant ainsi la disponibilité dudit métal - ou desdits métaux - de trace pour lesdits micro-organismes.

16. Méthode selon la revendication 15, dans laquelle le composé de liaison est de la pyrimine.

17. Méthode selon la revendication 15, dans laquelle ledit composé de liaison est un composé à base d'amine.

18. Méthode selon la revendication 15, dans laquelle le métal de trace est du fer ferrique et le composé de liaison a une constante de liaison pour le fer ferrique d'au moins 10³⁰ litres par mole, et une capacité à former, avec le fer ferrique, un complexe au moins 10 fois plus stable cinétiquement qu'avec la ferrioxamine B.

19. Méthode selon la revendication 3 ou l'une quelconque des revendications 4 à 7 se rapportant à la revendication 3, dans laquelle ledit matériau d'emballage (8) est un conteneur de distribution (8), l'action de liaison étant favorisée par un mouvement relatif entre le matériau (22) et ledit liant par le fait de la manipulation du conteneur (8).

20. Méthode selon les revendications 8, 10 ou 15, dans laquelle le matériau (22) qui est fluide, est mis en contact avec ladite substance active en passant sur une surface sur laquelle se trouve ladite substance active.

21. Méthode selon les revendications 8, 10 ou 15, dans laquelle l'activité de ladite substance active est améliorée par un mouvement relatif entre le matériau (22) et ladite substance active.

22. Matériau d'emballage (8) ayant à sa surface (10, 12, 14, 16) une substance essentiellement insoluble, immobilisée, inhibitrice de croissance, prévue pour être mise en contact avec un autre matériau (22) se présentant comme un milieu complexe comprenant un liquide, ladite substance ayant une composition permettant de réagir avec ledit autre matériau (22) pour inhiber la croissance des micro-organismes dudit autre matériau (22), caractérisé en ce qu'il y a plusieurs substances actives qui ne sont pas des enzymes.

23. Matériau d'emballage selon la revendication 22, dans lequel les substances actives sont choisies parmi le groupe constitué par les lectines, polysaccharides, anticorps, chélateurs métalliques et les composés à base d'amine.

24. Matériau d'emballage selon les revendications 21 ou 22, dans lequel lesdites substances actives comprennent un liant ayant une affinité de liaison avec des constituants spécifiques dudit autre matériau (22), afin de lier lesdits constituants spécifiques, ledit liant ayant une grande affinité de liaison avec lesdits constituants spécifiques et une faible affinité de liaison avec les autres constituants dudit autre matériau (22) qui tendent à inhiber la liaison avec lesdits constituants spécifiques.

25. Matériau d'emballage selon la revendication 24, dans lequel lesdits constituants spécifiques sont lesdits micro-organismes.

26. Matériau d'emballage selon les revendications 24 ou 25, dans lequel ledit liant est une lectine ou un composé à base d'amine.

27. Matériau d'emballage selon l'une quelconque des revendications 22 à 26, dans lequel une enzyme est associée auxdites substances actives.

28. Matériau d'emballage (8) ayant à sa surface (10, 12, 14, 16) une substance essentiellement insoluble, immobilisée, inhibitrice de croissance, prévue pour être mise en contact avec un autre matériau (22) se présentant comme un milieu complexe comprenant un liquide, ladite substance ayant une composition permettant de réagir avec ledit autre matériau (22) pour inhiber la croissance des micro-organismes dudit autre matériau (22), dans lequel ladite substance active comprend - ou lesdites substances actives comprennent - un composé à base d'amine, dont l'amine est prévue pour - et capable de - provoquer une destruction des micro-organismes dudit autre matériau (22) suffisante pour inhiber leur croissance dans ledit autre matériau (22).

29. Matériau d'emballage selon la revendication 28, dans lequel ledit composé est une amine tertiaire.

30. Matériau d'emballage (8) ayant à sa surface (10, 12, 14, 16) une substance essentiellement insoluble, immobilisée, inhibitrice de croissance, prévue pour être mise en contact avec un autre matériau (22) se présentant comme un milieu complexe comprenant un liquide, ladite substance ayant une composition permettant de réagir avec ledit autre matériau (22), pour inhiber la croissance de micro-organismes dans ledit autre matériau (22), dans lequel ladite substance active comprend - ou lesdites substances actives comprennent - un liant se présentant comme un composé de liaison ayant une affinité pour au moins un métal de trace constituant un facteur de croissance biologique et donnant une constante de liaison et une stabilité cinétique pour lier ledit métal - ou lesdits métaux - de trace, qui soient suffisantes pour que le composé de liaison réussisse à concurrencer les micro-organismes vis-à-vis dudit métal - ou desdits métaux - de trace, et pour retenir le métal - ou les métaux - de trace liés, restreignant ainsi la disponibilité dudit - ou desdits - facteur(s) de croissance pour lesdits micro-organismes.

31. Matériau d'emballage selon la revendication 30, dans lequel le composé de liaison est de la pyrimine.

32. Matériau d'emballage selon la revendication 30, dans lequel le composé de liaison présente une affinité de liaison pour le fer ferrique et une constante de liaison pour le fer ferrique d'au moins 10³⁰ litres par mole, et une capacité à former, avec le fer ferrique, un complexe au moins 10 fois plus stable cinétiquement qu'avec la ferrioxamine B.

33. Matériau d'emballage selon l'une quelconque des revendications 22 à 27, dans lequel lesdites substances actives sont disposées sur des zones distinctes de ladite surface (10, 12, 14, 16).

34. Matériau d'emballage selon l'une quelconque des revendications 22 à 27, dans lequel lesdites substances actives sont mélangées entres elles sur ladite surface (10, 12, 14, 16).

35. En association, un milieu complexe (22), un liant ayant une affinité de liaison avec les micro-organismes dudit milieu pour se lier auxdits micro-organismes, et un agent biocide de neutralisation desdits micro-organismes, ledit liant étant immobilisé sur une surface en contact avec ledit milieu complexe (22).

36. Un composé selon la revendication 35, dans lequel ledit liant est une lectine ou un composé à base d'amine.

37. Un composé selon la revendication 36, dans lequel ledit composé est une amine tertiaire.

38. Un composé selon l'une quelconque des revendications 35 à 37, dans lequel ledit agent biocide est un composé à base d'amine.

39. Utilisation d'une substance active permettant de détruire les micro-organismes d'un milieu complexe, suffisamment pour inhiber leur croissance dans ledit milieu, caractérisée en ce que ladite substance active comprend un composé à base d'amine immobilisé sur une surface en contact avec ledit milieu complexe.

40. Utilisation d'une substance active selon la revendication 39, dans laquelle ledit composé est une amine tertiaire.

41. Utilisation d'une substance active selon la revendication 39, dans laquelle ledit composé est une amine tertiaire partiellement oxydée.
